# Europäisches Patentamt

# European Patent Office

# Office européen des brevets

(19)

(11) Veröffentlichungsnummer: **0 091 063**
**B1**

(12) ## EUROPÄISCHE PATENTSCHRIFT

(45) Veröffentlichungstag der Patentschrift:
01.07.87

(21) Anmeldenummer: **83103078.8**

(22) Anmeldetag: **28.03.83**

(51) Int. Cl.⁴: **C 12 P 19/24,** C 12 P 19/12 //
C12N11/00

(54) **Verfahren zur Herstellung von Isomaltulose (6-0-alpha-D-Glucopyranosido-D-fructose) mit Hilfe von immobilisierten Bakterienzellen.**

(30) Priorität: **07.04.82 DE 3213107**

(43) Veröffentlichungstag der Anmeldung:
**12.10.83 Patentblatt 83/41**

(45) Bekanntmachung des Hinweises auf die Patenterteilung:
**01.07.87 Patentblatt 87/27**

(84) Benannte Vertragsstaaten:
**AT BE CH DE FR GB IT LI NL SE**

(56) Entgegenhaltungen:
EP-A-0 028 900
EP-A-0 049 742
EP-A-0 109 009
FR-A-2 179 966

**CHEMICAL ABSTRACTS, Band 80, Nr. 1, 7. Januar 1974, Seite 128, Nr. 1327a, Columbus, Ohio, USA. B.M. LUND et al.: "Nature of reducing compounds formed from sucrose by Erwinia carotovora var atroseptica"**

(73) Patentinhaber: **Süddeutsche Zucker-Aktiengesellschaft, Maximilianstrasse 10, D-6800 Mannheim 1 (DE)**

(72) Erfinder: **Munir, Mohammad, Dr., Wormser Strasse 11, D-6719 Obrigheim 1 (DE)**

(74) Vertreter: **Körber, Wolfhart, Dr., Patentanwälte Dipl.- Ing. H. Mitscherlich Dipl.- Ing. K. Gunschmann Dr.rer.nat. W. Körber Dipl.Ing. J. Schmidt- Evers Dipl.- Ing. W. Melzer Steinsdorfstrasse 10, D-8000 München 22 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

## Beschreibung

In der älteren Patentanmeldung P 30 38 219.5-41 (EP-A-49 742) ist vorgeschlagen, Isomaltulose mit Hilfe von Mikrooroanismen durch enzymatische Umwandlung der Saccharose herzustellen, in dem man reine Saccharoselösungen mit toten, immobilisierten Zellen von Isomaltulose aus Saccharose bildenden Mikroorganismen behandelt bzw. in Kontakt bringt. Dabei werden Saccharoselösungen im Konzentrationsbereich von 45 Gew.-% bis 75 Gew.-%, vorzugsweise 65 Gew.-% bis 75 Gew-%, bei einer Temperatur von 40°C bis 65°C kontinuierlich durch einen mit den immobilisierten Zellen gefüllten Reaktor geleitet. Die dabei entstandene Isomaltulose wird durch an sich bekannte Methoden kristallin gewonnen.

Bei den verwendeten Mikroorganismen handelt es sich um Protaminobacter rubrum (CBS 574.77), Serratia plymuthica (ATCC 15928), Serratia marcescens (NCIB 8285) und Leuconostoc mesenteroides (NRRL B-512 F (ATCC 10830a)), vorzugsweise um Protaminobacter rubrum (CBS 574.77).

Bei der Durchführung dieses vorgeschlagenen Verfahrens hat sich gezeigt, daß häufig ein hoher Anteil (bis zu rund einem Drittel) der eingesetzten Saccharose nicht in die gewünschte Verbindung (Isomaltulose) sondern in Nebenprodukte umgewandelt wird und/oder unumgesetzt in der Reaktionslösung verbleibt. Da diese Nebenprodukte auch die Kristallisation der entstandenen Isomaltulose hemmen und somit die Kristallausbeute noch weiter herabsetzen, ist es von großem wirtschaftlichen Interesse, ein Verfahren zu haben, das die Bildung von unerwünschten und störenden Nebenprodukten ganz oder wenigstens teilweise verhindert.

Es wurde nun überraschenderweise gefunden, daß es möglich ist, Isomaltulose in hoher Reinheit und Ausbeute aus Saccharose dadurch zu gewinnen, daß man die Saccharoselösung mit einer Konzentration von 40 Gew.-% bis 75 Gew.-%, vorzugsweise 45 Gew.-% bis 60 Gew.-%, in zwei Teile teilt, den ersten Teil über einen mit immobilisierten, toten Zellen von Isomaltulose aus Saccharose bildenden Mikroorganismen gefüllten Reaktor bei 25°C bis 40°C, vorzugsweise 30°C bis 40°C, so leitet, daß 80 - 90 % der Saccharose umgesetzt werden, den zweiten Teil der Saccharoselösung mit der Mutterlauge aus einer vorangegangenen Isomaltulose-Kristallisation mischt, diese Lösung über einen zweiten mit immobilisierten, toten Zellen von Isomaltulose aus Saccharose bildenden Mikroorganismen gefüllten Reaktor bie 25°C bis 40°C, vorzugsweise 30°C bis 40°C, leitet, so daß die Saccharose nahezu vollständig umgesetzt wird, die entstandene Isomaltulose aus dieser Lösung durch an sich bekannte Methoden kristallin gewinnt, die Kristalle in dem umgesetzten ersten Teil der Reaktionslösung auflöst und zur Kristallisation bringt, die entstandenen Isomaltulose-Kristalle abtrennt und die Mutterlauge zum Zumischen zu dem zweiten Teil der Saccharoselösung verwendet.

Die einzuhaltende mittlere Verweilzeit der Saccharoselösung im Reaktor richtet sich nach der jeweils vorhandenen spezifischen Aktivität der immobilisierten Zellen. Die spezifische Aktivität wird als μmol umgesetzte Saccharose pro g immobilisierte Zellen (trocken) pro Minute definiert. Beispielsweise enthält ein Reaktor mit 100 mm Durchmesser und 450 mm Betthöhe 950 g immobilisierte Zellen (trocken) mit einer spezifischen Aktivität von 60 Einheiten/g. Dieser Reaktor muß mit 1900 ml Saccharoselösung (50 Gew.-%) pro Stunde durchströmt werden um 90% der eingebrachten Saccharose umzusetzen. Da die Aktivität über die Betriebszeit kontinuierlich abnimmt, muß die Strömungsgeschwindigkeit ständig nachreguliert werden, um ein Produkt mit konstanter Zusammensetzung zu erhalten.

Die besonderen Vorteile des anmeldungsgemäßen Verfahrens werden anhand von folgenden Beispielen näher erläutert. Dabei stellt Beispiel 1 Arbeitsweise und Ergebnisse des bekannten Verfahrens der deutschen Patentanmeldung P 30 38 219.5-41 (= EP-A-4 97 42) dar.

## Beispiel 1

a) Von einer Abimpfung des Stammes Protaminobacter rubrum (CBS 574.77) werden Zellen mit 10 ml eines sterilen Nährsubstrates, bestehend aus 8 kg Dicksaft aus einer Zuckerfabrik (Trockensubstanzgehalt = 65%), 2 kg Maisquellwasser 0,1 kg $(NH_4)_2HPO_4$ und 89,9 kg dest. Wasser (bei Bedarf auf pH 7,2 eingestellt) abgeschwemmt. Diese Suspension dient als Impfgut für die Schüttelmaschinen-Vorkultur in 1-1-Kolben mit 200 ml Nährlösung obiger Zusammensetzung.

Nach einer 30-stündigen Bebrütungszeit bei 29°C werden 16 l Nährlösung obiger Zusammensetzung mit 20 Kolben (Gesamtinhalt 4 l) in einem 30-l-Klein-fermenter beimpft und bei derselben Temperatur bei einer Belüftungsrate von 20 l Luft pro Minute und einer Rührgeschwindigkeit von 350 UpM fermentiert. Die anwachsende Keimzahl wird mikroskopisch bestimmt. Nach Erreichen von $5.10^9$ Keimen/ml wird der Fermenterinhalt in ein anderes Gefäß transferiert, dort mit einem kationenaktiven Flockungsmittel (wie z. B. PRIMAFLOC C7® von Rohm & Haas, Philadelphia/USA) versetzt. Die ausgeflockten Zellen werden absetzen gelassen, abdekantiert, mit 0,1 M Phosphatpuffer (pH 7,0) gewaschen und an der Zentrifuge entwässert. Die Masse wird dann zu Strängen extrudiert, an der Luft getrocknet und gemahlen.

Die Siebfraktion 0,3 bis 0,8 mm aus dem oben gewonnenen Präparat wird in einer 0,1%-igen Glutaraldehydlösung 10 Minuten gerührt, mit Phosphatpuffer (0,1M, pH 7,0) gewaschen und unter Wasser in eine temperierbare Säule gefüllt. Die Säule wird dann auf 50°C erwärmt und mit einer 70 gew.-%-igen Saccharoselösung kontinuierlich durchströmt. Die Strömungsgeschwindigkeit wird dabei so eingestellt, daß am Ende der Säule keine Saccharose mehr nachweisbar ist. Die so gewonnene Isomaltuloselösung hat folgende durchschnittliche Zusammensetzung (HPLC = Hochdruck-Flüssigkeitschromatographie):

| | |
|---|---|
| Fructose | 7,4 g/100 g TS |
| Glucose | 0,3 g/100 g TS |
| Saccharose | 0,1 g/100 g TS |
| Isomaltulose | 62,6 g/100 g TS |
| 1-0-α-D-Glucopyrano-sido-D-Fructose | 16,6 g/100 g TS |
| Oligosaccharide | 13,0 g/100 g TS |

Diese Lösung wird einem Kühlungskristallisator zugeführt, auf 20°C gekühlt und die auskristallisierte Isomaltulose an Siebkorbzentrifugen von der Mutterlauge abgetrennt. Die Mutterlauge wird auf 78 - 82 % TS-Gehalt eingedampft, einer 2. Kühlungskristallisation bis auf 20°C unterworfen und die auskristallisierte 2. Isomaltulosemenge an Siebkorbzentrifugen von der Mutterlauge abgetrennt.

Ausbeute:

100 kg einer Saccharoselösung mit 70 kg Saccharose enthalten nach der Umsetzung 43,8 kg Isomaltulose (62,6 % v. TS-Gehalt). Daraus werden bei der 1. Kristallisation 15,9 kg reine Isomaltulose und bei der 2. Kristallisation 13,4 kg Isomaltulose mit 98 % Reinheit gewonnen. Das entspricht einer Gesamtausbeute von 29 kg reiner Isomaltulose aus 70 kg Saccharose oder 41,4 % vom Rohstoff. (In diesem Beispiel und in den nachfolgenden wird mit wasserfreier Isomaltulose gerechnet.)

**Beispiel 2**

Die Siebfraktion 0,3 bis 0,8 mm aus dem nach Beispiel 1 a) hergestellten Präparat wird in einer 0,1 %-igen Glutaraldehydlösung 10 Minuten gerührt, mit Phosphatpuffer (0,1M, pH 7,0) gewaschen und unter Wasser in eine temperierbare Säule gefüllt. Die Säule wird dann auf 30°C erwärmt und mit einer 50 gew.-%-igen Saccharoselösung kontinuierlich durchströmt. Die Strömungsgeschwindigkeit wird dabei so eingestellt, daß am Ende der Säule keine Saccharose mehr nachweisbar ist. Die so gewonnene Isomaltuloselösung hat folgende durchschnittliche Zusammensetzung (HPLC):

| | |
|---|---|
| Fructose | 3,6 g/100 g TS |
| Glucose | 1,6 g/100 g TS |
| Saccharose | 0 |
| Isomaltulose | 78,4 g/100 g TS |
| 1-0-α-D-Glucopyrano-sido-D-fructose | 12,6 g/100 g TS |
| Oligosaccharide | 3,6 g/100 g TS |

Diese Lösung wird auf 78 - 82 % TS-Gehalt eingedampft, einem Kühlungskristallisator zugeführt, auf 20°C gekühlt und die auskristallisierte Isomaltulose an Siebkorbzentrifugen von der Mutterlauge abgetrennt. Die Mutterlauge wird wiederum auf 78 - 82 % TS-Gehalt eingedampft, einer 2. Kühlungskristallisation bis auf 20°C unterworfen und die auskristallisierte 2. Isomaltulosemenge an Siebkorbzentrifugen von der Mutterlauge abgetrennt.

Ausbeute:

100 kg einer Saccharoselösung mit 50 kg Saccharose enthalten nach der Umsetzung 39,2 kg Isomaltulose (78,4 % v. TS-Gehalt). Daraus werden bei der 1. Kristallisation 25,0 kg reine Isomaltulose und bei der 2. Kristallisation 9,6 kg Isomaltulose mit 98 % Reinheit gewonnen. Das entspricht einer Gesamtausbeute von 34,4 kg reiner Isomaltulose aus 50 kg Saccharose oder 68,8 % vom Rohstoff.

**Beispiel 3**

Die Siebfraktion 0,3 bis 0,8 mm aus dem nach Beispiel 1 a) hergestellten Präparat wird in einer 0,1 %-igen Glutaraldehydlösung 10 Minuten gerührt, mit Phosphatpuffer (0,1M, pH 7.0) gewaschen und unter Wasser in zwei auf 30°C temperierten Säulen gefüllt.

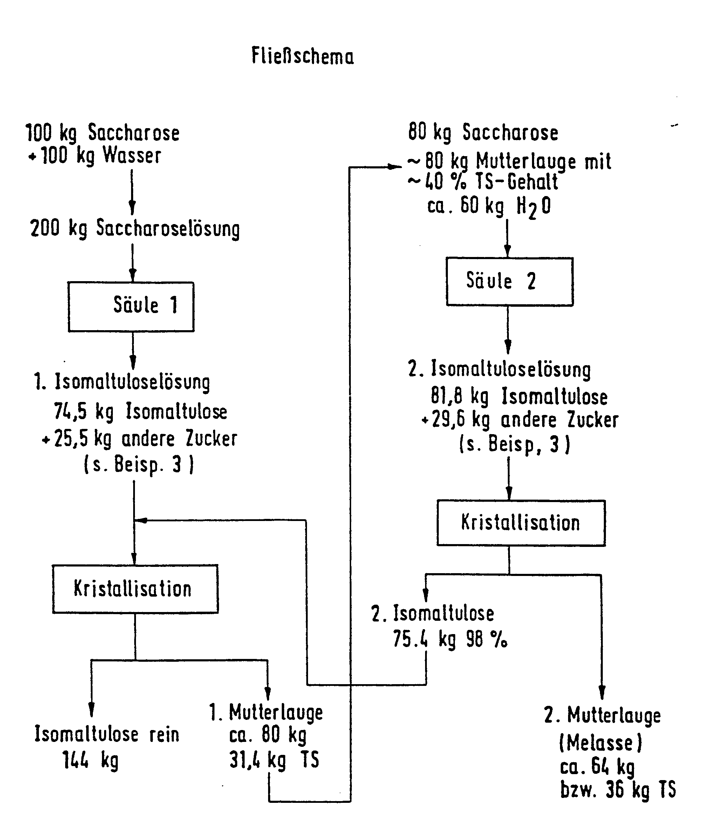

Die Arbeitsweise wird anhand des Schemas (Fig. 1) beispielhaft erläutert.

Eine der Säulen (1) wird mit einer 50 gew.-%-igen Saccharoselösung kontinuierlich durchströmt. Die Strömungsgeschwindigkeit wird dabei so eingestellt, daß beim Durchströmen der Säule nur etwa 90 % der Saccharose umgesetzt werden. Die so erhaltene Reaktionslösung hat folgende beispielhafte Zusammensetzung (HPLC):

| | |
|---|---|
| Fructose | 2,9 g/100 g TS |

0 091 063

| Glucose | 1,6 g/100 g TS |
|---|---|
| Saccharose | 10,8 g/100 g TS |
| Isomaltulose | 75,2 g/100 g TS |
| 1-0-α-D-Glucopyrano-sido-D-fructose | 6,4 g/100 g TS |
| Oligosaccharide | 3,1 g/100 g TS |

Parallel zu der ersten Säule wird die zweite Säule (2) mit einem Substrat beschickt, das aus einem Gemisch aus Saccharose und Mutterlauge einer vorangegangenen Isomaltulose-Kristallisation besteht. Eine beispielhafte Zusammensetzung (HPLC) dieses Substrates ist:

| TS-Gehalt | 50 Gew.-% |
|---|---|
| Fructose | 2,0 g/100 g TS |
| Glucose | 1,0 g/100 g TS |
| Saccharose | 76,2 g/100 g TS |
| Isomaltylose | 10,8 g/100 g TS |
| 1-0-α-D-Glucopyrano-sido-D-fructose | 8,2 g/100 g TS |
| Oligosaccharide | 1,8 g/100 g TS |

Die Fließgeschwindigkeit durch diese Säule wird so eingestellt, daß die Lösung am Ende der Säule höchstens 1 g Saccharose pro 100 g Trockensubstanz enthält. Die so erhaltene Reaktionslösung hat folgende beispielhafte Zusammensetzung (HPLC):

| Fructose | 5,4 g/100 g TS |
|---|---|
| Glucose | 2,2 g/100 g TS |
| Saccharose | 1,0 g/100 g TS |
| Isomaltulose | 72,3 g/100 g TS |
| 1-0-α-D-Glucopyrano-sido-D-fructose | 16,6 g/100 g TS |
| Oligosaccharide | 2,5 g/100 g TS |

Diese Lösung wird auf 78 - 82 % TS-Gehalt eingedampft, einer Kühlungskristallisation bis auf 20°C unterworfen und die auskristallisierte Isomaltulose an Siebkorbzentrifugen von dieser 2. Mutterlauge abgetrennt.

Die an dieser Stelle erhaltene Isomaltulose wird in der von der ersten Säule erhaltenen Reaktionslösung gelöst, auf 78 - 82 % TS-Gehalt eingedampft, in einen Kühlungskristallisator auf 20°C gekühlt und die auskristallisierte reine Isomaltulose an Siebkorbzentrifugen von der Mutterlauge abgetrennt. Die an dieser Stelle anfallende Mutterlauge wird für die Substratherstellung für die zweite Säule verwendet.

Die 2. Mutterlauge, die auch als Melasse bezeichnet werden kann, enthält ca. 30 - 40 % a. TS an 1-0-α-Gluco-pyranosido-D-fructose und kann als Rohstoff für die Gewinnung von diesem Zucker eingesetzt werden. Die winnung dieses Zuckers kann zum Beispiel durch chromatographische Trennung an Ionenaustauscher oder anderen geeigneten Trennmaterialien, nach einer Voranreicherung durch Auskristallisieren eines weiteren Teiles der Isomaltulose aus Methanol und Entfernen der durch Hefe vergärbaren Zucker durch Behandlung mit freier oder immobilisierter Hefe, durchgeführt werden.

Ausbeute:

Bei der in diesem Beispiel geschilderten Arbeitsweise gewinnt man pro 100 kg Saccharose 80,0 kg reine Isomaltulose.

**Patentansprüche**

1. Verfahren zur Herstellung von Isomaltulose (6-0-α-D-Glucopyranosido-D-fructose) durch enzymatische Umwandlung aus Saccharose mit Hilfe von Isomaltulose aus Saccharose bildenden Mikroorganismen, bei dem man reine Saccharoselösungen mit toten, immobilisierten Zellen solcher Mikroorganismen behandelt bzw. in Kontakt bringt und die entstandene Isomaltulose durch Auskristallisieren gewinnt, dadurch gekennzeichnet, daß man zunächst einen Teil der Saccharoselösung mit einer Konzentration von 40 Gew.-% bis 75 Gew.-%, vorzugsweise 45 Gew.-% bis 60 Gew.-%, über einen mit den immobilisierten, toten Zellen gefüllten Reaktor bei 25°C bis 40°C, vorzugsweise 30°C bis 40°C, leitet, so daß 80 - 90 % der Saccharose umgesetzt werden, die nach dem Auskristallisieren der gebildeten Isomaltulose anfallende erste Mutterlauge mit einem weiteren Teil der Saccharoselösung über einen zweiten, ebenfalls mit den immobilisierten, toten Zellen gefüllten Reaktor bei 25°C bis 40°C, vorzugsweise 30°C bis 40°C, leitet, so daß die Saccharose nahezu vollständig umgesetzt wird, die aus diesem zweiten Teil der Lösung entstandene Isomaltulose durch an sich bekannte Methoden kristallin gewinnt, die Kristalle in den umgesetzten ersten Teil der Lösung auflöst, zur Kristallisation bringt und die entstandenen Isomaltulose-Kristalle abtrennt.

2. Verfahren nach Anspruch 1, dadurch gekennzeichnet, daß es sich bei den verwendeten Mikroorganismen um Protaminobacter rubrum (CBS 574.77), Serratia plymuthica (ATCC 15928), Serratia marcescens (NCIB 8285) und Leuconostoc mesenteroides (NRRL B-512 F (ATCC 10830a)), vorzugsweise um Protaminobacter rubrum (CBS 574.77), handelt.

3. Verfahren zur Gewinnung von 1-0-α-Glucopyranosido-D-fructose nach Anspruch 1, dadurch gekennzeichnet, daß man aus einer Mutterlauge der Isomaltulose-Gewinnung nach Voranreicherung durch Auskristallisieren eines weiteren Teiles der Isomaltulose aus Methanol und Entfernen der durch Hefe vergärbaren Zucker durch Behandlung mit freier oder immobilisierter Hefe die 1-0-α-Gluco-pyranosido-D-fructose durch chromatographische Trennung an Ionenaustauschern oder anderen geeigneten Trennmaterialien zunächst als wässerige Lösung gewinnt und dann durch an sich bekannte Methoden in trockene Form überführt.

## Claims

1. In a process for preparing isomaltulose (6-0-α-D-glucopyranosido-D-fructose) wherein sucrose solution is treated with or brought into contact with dead immobilized cells of a microorganism and the formed isomaltulose is obtained by crystallizing characterized in passing one part of the sucrose solution having a concentration of 40 % by weight to 75 % by weight, preferably 45 % by weight to 60 % by weight via a reactor, which is filled with immobilized dead cells at 25° C to 40°, preferably 30° C to 40° C so that 80 % to 90 % of the sucrose is converted, in passing the first mother liquor obtained after having crystallized the formed isomaltulose with a further part of the sacrose solution via a second reactor, which is also filled with immobilized, dead cells at 25° C to 40° C, preferrably 30° C to 40° C so that the sucrose is almost completely converted, and by obtaining the isomaltulose resulting from the second part of the solution in crystalline form by methods known per se, in dissolving and crystallizing the crystals in the converted first part of the solution and in separating the resultant isomaltulose crystals.

2. Process of claim 1, wherein said microorganism is selected from the group consisting of Protaminobacter rubrum (CBS 574.77), Serratia plymuthica (ATCC 15928), Serratia marcescens (NCIB 8285), and Leuconostoc mesenteroides (NRRL B-512 F (ATCC 10830a)), preferably Protaminobacter rubrum (CBS 574.77).

3. A process for obtaining 1-0-α-glucopyranosido-D-fructose according to claim 1, characterized in that from the mother liquor of the isomaltulose obtained after cobbing by crystallizing a further part of the isomaltulose from methanol, removing sugar fermentable by yeast by treatment with free or immobilised yeast, the 1-0-α-glucopyranosido-D-fructose can be obtained by chromatographic separation by means of ion exchangers or other suitable separating material firstly as an aqueous solution and then transformed to dry form by methods known per se.

## Revendications

1. Procédé pour la préparation d'isomaltulose (6-0-alpha-D-glucopyranosido-D-fructose) par transformation enzymatique a partir de saccharose à l'aide d'isomaltulose provenant de microorganismes produisant de la saccharose, dans lequel on traite et/ou met en contact des solutions pures de saccharose avec des cellules immobilisées mortes de tels microorganismes et on obtient l'isomaltulose par cristallisation, caractérisé en ce qu'on fait passer d'abord une partie de la solution de saccharose a une concentration de 40 à 75 % en poids et de preférence de 45 à 60 %, par un reacteur rempli de cellules mortes et immobilisées, à une température de 25 à 40° C et de préférence de 30 à 40° C, de sorte que 80 à 90 % de la saccharsoe entre en reaction; en ce qu'on fait passer la première liqueur mère produite après la cristallisation de l'isomaltulose formée, avec une autre partie de la solution de saccharose par un deuxième reacteur, rempli également de cellules mortes et immobilisées, à une temperature de 25 à 40° C et, de préférence, de 30 à 40° C, de sorte que la saccharose entre presque complètement en reaction; en ce que l'isomaltulose formée a partir de cette deuxième partie de la solution est cristallisée par des methodes connues et en ce qu'on dissout les cristaux dans la première partie - entrée en réaction - de la solution, on provoque une cristallisation et on separe les cristaux d'isomaltulose obtenus.

2. Procédé suivant la revendication 1, caractérisé en ce que les microorganismes utilisés sont le protaminobacter rubrum (CBS 574.77), la serratia plymuthica (ATCC 15 928), la serratia marcescens (NCIB 8285) et le leuconostoc mesenteroides (NRRL B-512 F (ATCC 10830a)), et de préférence le protaminobacter rubrum (CBS 574.77).

3. Procédé pour l'obtention de 6-0-alpha-D-glucopyranosido-D-fructose suivant la revendication 1, caractérisé en ce qu'a partir d'une lessive mère pour l'obtention d'isomaltulose, après enrichissement préalable par cristallisation d'une autre partie de l'isomaltulose provenant du méthanol et élimination de sucre à fermenter par levure, par traitement avec de la levure libre ou immobilisée, le 6-0-alpha-D-glucopyranosido-D-fructose est obtenu d'abord sous forme de solution aqueuse par séparation chromatographique sur des échangeurs d'ions ou d'autres substances appropriées, puis seché par des méthodes connues.

Fließschema

100 kg Saccharose
+ 100 kg Wasser

200 kg Saccharoselösung

**Säule 1**

1. Isomaltuloselösung
74,5 kg Isomaltulose
+ 25,5 kg andere Zucker
(s. Beisp. 3)

**Kristallisation**

Isomaltulose rein
144 kg

1. Mutterlauge
ca. 80 kg
31,4 kg TS

80 kg Saccharose
~ 80 kg Mutterlauge mit
~ 40 % TS-Gehalt
ca. 60 kg $H_2O$

**Säule 2**

2. Isomaltuloselösung
81,8 kg Isomaltulose
+ 29,6 kg andere Zucker
(s. Beisp. 3)

**Kristallisation**

2. Isomaltulose
75.4 kg 98 %

2. Mutterlauge
(Melasse)
ca. 64 kg
bzw. 36 kg TS

1